# EUROPEAN PATENT APPLICATION

(11) **EP 3 061 830 A1**
(43) Date of publication of application: **31.08.2016**
(21) Application number: 14854911.6
(22) Date of filing: 16.10.2014
(51) Int. Cl.: C12Q 1/68, C12N 15/09, G01N 1/02

(54) **METHOD FOR TESTING FOR MICROORGANISMS**

(30) Priority: 22.10.2013 JP 2013218854
(71) Applicant: Toyo Seikan Group Holdings, Ltd., Shinagawa-ku Tokyo 141-8627 (JP)
(72) Inventor: OTSU, Takayoshi, Yokohama-shi Kanagawa 240-0062 (JP); ISSHIKI, Atsunori, Yokohama-shi Kanagawa 240-0062 (JP)
(74) Representative: Pestalozzi, Deborah
(86) International application number: PCT/JP2014/005255
(87) International publication number: WO 2015/059907

(57) **Abstract**

To enable further shortening of the entire testing period including sampling in a method for testing a microorganism. Provided is a method for testing a microorganism to simultaneously judge the presence or absence of plural types of microorganisms in the environment, which includes: a sampling step for collecting microorganisms floating in the air in a liquid; a nucleic acid extraction step for extracting nucleic acids from the microorganisms in the liquid; a nucleic acid amplification step for amplifying part of the nucleic acids extracted; and a detection step for dropping a solution containing the amplified nucleic acids to a carrier on which nucleic acids of plural types of microorganisms have been immobilized in advance and identifying the types of microorganisms when nucleic acids that bind complementary with the amplified nucleic acids are immobilized on the carrier, wherein culturing of the microorganisms is not conducted after the sampling step and before the nucleic acid extraction step.

SEQUENCE LISTING

<110>  TOYO SEIKAN GROUP HOLDINGS, LTD.

<120>  METHOD FOR DETECTING MICROORGANISM

<130>  TSK-1465-PCT

<160>  8

<170>  PatentIn version 3.5

<210>  1
<211>  26
<212>  DNA
<213>  Artificial

<220>
<223>  synthetic DNA

<400>  1
ttggtcattt agaggaagta aaagtc                                          26


<210>  2
<211>  19
<212>  DNA
<213>  Artificial

<220>
<223>  synthetic DNA

<400>  2
ctgcgttctt catcgatgc                                                  19


<210>  3
<211>  24
<212>  DNA
<213>  Artificial

<220>
<223>  synthetic DNA

<400>  3
ggtaaccaaa tcggtgctgc tttc                                            24


<210>  4
<211>  24
<212>  DNA
<213>  Artificial

<220>
<223>  synthetic DNA

<400>  4
accctcagtg tagtgaccct tggc                                            24


<210>  5
<211>  21
<212>  DNA

<213>  Aspergillus penicillioides

<400>  5
gagacctcaa ccatgaacac t                                                21


<210>  6
<211>  22
<212>  DNA
<213>  Aspergillus penicillioides

<400>  6
catcgtcagc atgtcacacc gc                                               22


<210>  7
<211>  23
<212>  DNA
<213>  Cladosporium sp.

<400>  7
actcttgcgt aactttgcag tct                                              23


<210>  8
<211>  23
<212>  DNA
<213>  Wallemia sebi

<400>  8
ccaagcagtt aagctgttga aat                                              23

## Description

### Technical Field

The present invention relates to a method for testing microorganisms. In particular, the present invention relates to a testing method for detecting plural types of microorganisms rapidly and simultaneously.

### Background Art

In recent years, in food manufacturing sites, clinical sites, environments for protecting cultural assets or the like, it has become important to check the presence or absence of microorganisms such as fungi to confirm safety, as well as to prevent the proliferation thereof.

As the method for testing microorganisms, in particular, fungi, the following three methods can be given.

As the first method, a method can be given in which specimens are obtained by sampling from the environment and the obtained specimens are then pre-cultured (simultaneous culturing of the collected fungi), and culturing is conducted for about 20 days in an optimum culture medium according to the type of fungus, and then the morphological characteristics are observed to identify and confirm the presence or absence of a microorganism (see Patent Document 1).

As the second method, a method can be given in which specimens are obtained by sampling from the environment and the obtained specimens are pre-cultured, each fungus is cultured separately, DNA is extracted from cultured cells, a target region is amplified by a PCR (polymerase chain reaction) method, and the base sequence of this region is analyzed, whereby microorganisms are identified and the presence or absence thereof is confirmed.

As the third method, a method can be given in which a probe formed of a base sequence that complementarily bonds to a target region is prepared and immobilized on the substrate of a DNA chip, an amplified product obtained by a PCR method is dropped onto the DNA chip to hybridize the target region with the probe, whereby microorganisms contained in a specimen are identified and the presence or absence thereof is confirmed (see Patent Document 2).

In the first method in which identification of a microorganism is conducted based on its morphology, it is required to conduct culturing separately after the microorganism is grown by pre-culturing. At this time, in order to allow morphological characteristics of the microorganism to be developed, it is necessary to prepare an optimum culture medium in accordance with the type of fungus and conduct culturing for a long period of time. Therefore, some types of a microorganism require a testing period of as long as about 50 days. Further, proficiency is required for identification of a microorganism, and hence, this method is not suited to a quick testing or a simple testing.

In the second method in which sequence analysis is conducted, it is required to culture separately according to the type of fungus after the pre-culturing. Therefore, a testing period of about 14 days is required. Further, since microorganisms are analyzed separately according to the type of fungus, this method is not suited to a case where a large number of specimens are required to be tested simultaneously.

On the other hand, in the third method in which a DNA chip is used, it is possible to detect microorganisms without conducting culturing separately in accordance with the type of fungus, and hence, the testing period can be shortened to about 9 days.

However, for those who actually conduct a testing, there is a demand for further shortening the testing period; specifically, they want to know the results of testing on the same day when a specimen is obtained by sampling from the environment.

However, in the third method in which a DNA chip is used, pre-culturing is also required, and about 7 days are taken for this pre-culturing. The third method cannot satisfy the demand for shortening the testing period.

Under such circumstances, a method for rapidly detecting a minute organism that enables a testing of a microorganism using a DNA chip without conducting pre-culturing has been proposed (see Patent Document 3).

Patent Document 1: JP-A-2007-195454
Patent Document 2: JP-A-2008-35773
Patent Document 3: JP-A-2007-508811

### Summary of the Invention

### Problems to be solved by the Invention

This method for rapidly detecting a minute organism is a method in which RNA is extracted from a microorganism. However, if the microorganism is in the form of a spore, RNA cannot be obtained. On the other hand, a microorganism in a non-spore state cannot be captured selectively. Therefore, a problem arises that a recovering step for allowing a spore to be germinated becomes essential.

Further, since this method enables detection of a microorganism without conducting an amplification of RNA or DNA complementary thereto, cells of a significantly large amount of microorganisms are required to be captured (in the Examples, 1,000,000 cells were used. Under a relatively contaminated environment, several days are required for sampling), and hence, there is a problem that shortening of the entire testing period including sampling is difficult.

The present invention has been made in view of the above-mentioned circumstances, and is aimed at providing a method for testing a microorganism that can further shorten the entire testing period including sampling.

### Means for Solving the Problem

In order to attain the above-mentioned object, the method for testing a microorganism according to the present invention is a method for testing a microorganism to simultaneously judge the presence or absence of plural types of microorganisms in the environment, which comprises:
a sampling step for collecting microorganisms floating in the air in a liquid;
a nucleic acid extraction step for extracting nucleic acids from the microorganisms in the liquid;
a nucleic acid amplification step for amplifying part of the nucleic acids extracted; and
a detection step for dropping a solution containing the amplified nucleic acids to a carrier on which nucleic acids of plural types of microorganisms have been immobilized in advance and identifying the type of microorganisms when nucleic acids that bind complementary with the amplified nucleic acids are immobilized on the carrier, wherein
culturing of the microorganisms is not conducted after the sampling step and before the nucleic acid extraction step.

### Advantageous Effects of the Invention

According to the present invention, it is possible to provide a method for testing a microorganism that can further shorten the entire testing period including sampling.

### Brief Description of the Drawings

Fig. 1 is a table and a graph showing the relationship between the amount of air collected by an air sampler and the number of fungi in the Examples;
Fig. 2 is a table showing the base sequence of a primer used in the Examples;
Fig. 3 is a table showing the base sequence of a probe used in the Examples; and
Fig. 4 is a table showing the results of detection (S/N ratio) by a DNA chip in the Examples.

### Mode for Carrying out the Invention

Hereinbelow, preferred embodiments of the present invention will be explained in detail.

The method for testing a microorganism according to the embodiment of the present invention is a method for testing a microorganism to simultaneously judge the presence or absence of plural types of microorganisms in the environment, which comprises:
a sampling step for collecting microorganisms floating in the air in a liquid;
a nucleic acid extraction step for extracting nucleic acids from the microorganisms in the liquid;
a nucleic acid amplification step for amplifying part of the nucleic acids extracted; and
a detection step for dropping a solution containing the amplified nucleic acids to a carrier on which nucleic acids of plural types of microorganisms have been immobilized in advance and identifying the types of microorganisms when nucleic acids that bind complementary with the amplified nucleic acids are immobilized on the carrier, wherein
culturing of the microorganisms is not conducted after the sampling step and before the nucleic acid extraction step.

The method for testing a microorganism of this embodiment can include the following steps:

### (1) Sampling step

In the sampling step, microorganisms floating in the air are collected in a liquid. At this time, as a sampling apparatus for collecting microorganisms, a cyclonic air sampler is preferably used. As a liquid, in order to enhance the efficiency of collecting microorganisms, it is preferable to use a liquid containing a surfactant.

By using a cyclonic air sampler, air is helically incorporated into the liquid in the sampling apparatus at a high speed, and then discharged. During that process, fine particles in the air are attached to the inner wall of the sampling apparatus by centrifugal force. As a result, it is possible to collect in the liquid in the sampling apparatus the microorganism in the air.

As the cyclonic air sampler, Coriolis µ (liquid phase centrifugation method: liquid cyclone, manufactured by Bertin Technologies, Co., Ltd.) can be preferably used, for example.

If a cyclonic air sampler is used, it is preferable that air suction be conducted at a flow rate of 300 Umin for 1 to 10 minutes.

Due to the use of a cyclonic air sampler in the sampling step, a relatively large amount of microorganisms can be collected from a large volume of air for a short period of time. Therefore, even if culturing (pre-culturing) of a microorganism is not conducted prior to the nucleic acid extraction step, a nucleic acid of a microorganism can be sufficiently amplified in the nucleic acid amplification step, and the microorganism can be detected in the detection step.

Further, since the pre-culturing of a microorganism is not required to be conducted, a risk can be reduced that the microorganism is not detected since appropriate growth of the microorganism is inhibited by the conditions of a culture medium.

In the method for testing a microorganism in this embodiment, the "microorganism" includes true fungi such as fungi and yeast, bacteria such as food poisoning bacteria and other small organisms that cannot be seen unless a microscope is used.

In particular, according to the testing method of this embodiment, detection of fungi in the form of a spore or mycelia and floating in the air becomes possible. Such detection is important in food inspection, epidemiological environmental testing, environmental testing, clinical studies, livestock hygiene, or the like.

In addition, since culturing of a microorganism is not required to be conducted, it is possible to identify the type of a microorganism not only from a living microorganism but also from a corpse of a microorganism such as dead bacteria.

### (2) Nucleic acid extraction step

In the nucleic acid extraction step, a nucleic acid is extracted from a microorganism in the liquid obtained in the sampling step. As a result, genomic DNA can be obtained from a microorganism. A method of extracting a nucleic acid is not particularly limited and can be conducted by a normal method.

For example, by using a commercially available nucleic acid extraction kit (Water RNA/DNA Purification Kit-0.22 µm, manufactured by Norgen Biotek Corporation), a liquid is filtered by means of a filter to concentrate a microorganism, cells of the microorganism are crushed and then subjected to bacteriolysis, and a nucleic acid is purified, whereby extraction of a nucleic acid can be conducted preferably. In addition, extraction of a nucleic acid may be conducted by a CTAB (Cetyl trimethyl ammonium bromide) method or by using a DNA extraction apparatus.

### (3) Nucleic acid amplification step

In the nucleic acid amplification step, part of the nucleic acid extracted in the nucleic acid extraction step is amplified. As a result, it is possible to amplify a region to be amplified in genomic DNA as a specimen can be amplified to a number required for detection.

As mentioned above, according to a method for testing a microorganism in this embodiment, in the sampling method, by using a cyclonic air sampler, it is possible to collect in a liquid microorganisms floating in the air, and is possible to amplify nucleic acids of microorganisms in the nucleic acid amplification step.

Therefore, even if collection of a microorganism is not conducted for a long time in the sampling step, a nucleic acid of the microorganism can be sufficiently amplified in the nucleic acid amplification step. Therefore, it is possible to detect the microorganism appropriately in the detection step.

The method for amplifying nucleic acids is not particularly limited. However, a PCR method can be preferably used, for example.

It is preferred that a reaction liquid for PCR used in the PCR method have the following composition, for example. That is, a reaction liquid for PCR containing a nucleic acid synthesis substrate, a primer set, a nucleic acid synthesis enzyme, a labelling component, a genomic DNA of a specimen, a buffer solution and water (in an amount corresponding to the remainder of the volume) can be preferably used.

In the PCR method, by using a primer set for amplifying a region to be amplified in a genomic DNA of a specimen, the region to be amplified is amplified, whereby an amplified product can be obtained. Further, in the later detection step, the amplified product is hybridized with a probe that can bind complementarily with a part of the region to be amplified. Then, by detecting labelling components contained in the hybridized amplified product, a microorganism containing the genomic DNA can be identified.

As the buffer, Ampdirect (R) (manufactured by Shimadzu Corporation) can be used, for example.

As the PCR apparatus, a common thermal cycler or the like can be used.

As the PCR reaction conditions, the following conditions are preferable.
(a) 95°C 10 minutes, (b) 95°C (DNA denaturation step) 30 seconds, (c) 56°C (annealing step) 30 seconds, (d) 72°C (DNA synthesis process) 60 seconds ((b) to (d) 40 cycles), (e) 72°C 10 minutes

### (4) Detection step

In the detection step, a solution containing an amplified product obtained by the PCR method is dropped to a carrier on which probes of plural microorganisms are immobilized. If a probe complementarily bonds to the amplified product is immobilized on this carrier, the amplified product and the probe are hybridized. Then, by detecting labelling components contained in the hybridized amplified product, the types of the plural types of microorganisms can be independently identified specifically.

As the carrier, a DNA chip can be preferably used. As for a DNA chip, one obtained by the following method may be used; specifically, in a genomic DNA of a microorganism to be detected, a probe selected from a region to be amplified by the PCR method is synthesized in advance and immobilized on a substrate. Other configurations of the DNA chip are not particularly restricted as long as it is obtained by this method. For example, a spot type DNA chip, a synthetic DNA chip or the like can be used.

Specifically, detection of labelling components of the amplified product can be conducted as follows.

First, a prescribed buffer solution is mixed with an amplified product, and the resultant is dropped to a DNA chip. The DNA chip is allowed to stand at 45°C for 1 hour. Thereafter, by using a prescribed buffer solution, a PCR product that has not been hybridized is washed away from the DNA chip.

Subsequently, the DNA chip is mounted in a label detector to conduct detection of labelling components, and judges whether a microorganism to be detected is present or not. As the label detector, a common detector such as a fluorescence scanning apparatus can be used. Labels and the method for detecting thereof are not limited to those using fluorescence, and other methods can be used.

As explained above, according to the method for testing a microorganism in this embodiment, it is possible to collect in a liquid microorganisms floating in the air by using a cyclonic air sampler, and to amplify a nucleic acid of a microorganism in the nucleic acid amplification step. Therefore, without conducting pre-culturing that was an essential step to be conducted prior to the nucleic acid extraction step, a nucleic acid can be obtained in an amount sufficient for detection. As a result, plural types of microorganisms can be specifically detected simultaneously in the detection step.

In addition, even if collection of a microorganism is not conducted for a long period of time in the sampling step, a nucleic acid of a microorganism can be sufficiently amplified in the nucleic acid amplification step, microorganisms can be appropriately detected in the detection step.

Therefore, according to the method for testing microorganisms in this embodiment, the entire testing period including sampling can be further shortened.

In addition, according to the method for testing a microorganism in this embodiment, since a microorganism can be detected without conducting pre-culturing of the microorganism, it is also possible to identify a microorganism from a corpse of a microorganism floating in the air. Even in the case of a corpse of a microorganism, if inhaled by a human being, they might affect the health of the human being. Therefore, it is advantageous to enable detection of a corpse of a microorganism more rapidly than ever.

### EXAMPLES

Hereinbelow, a detailed explanation will be given on the Examples of the method for testing a microorganism according to the present invention.

In the sampling step, in order to collect in a liquid a microorganism floating in the air by using a cyclonic air sampler (Coriolis p, manufactured by Bertin Technologies, Co., Ltd.), air at two locations was suctioned and four samples in total were obtained as follows. The flow rate of the air sampler was fixed at 300 L/min.

First, suction of air was conducted three times at location A (in a rest room), and collection was conducted for 1 minute, 3 minutes and 5 minutes, thereby to obtain samples 1 to 3. For the samples 1 to 3, the amounts of air collected were 300 L, 900 L and 1500 L, respectively.

At location B (in a reception room), suction of air was conducted once. Collection time was 10 minutes to obtain sample 4. For the sample 4, the amount of air collected was 3000L.

The amount of a liquid for collecting a microorganism was 10 mL for all samples at the time when collection of air was started. After collecting air, the amount of the liquid is reduced by evaporation of the sample during collection. Therefore, the longer the collection time is, the smaller the amount of the remaining liquid is. For all samples, 5.1 mL or more of the liquid remained.

From the liquids of samples 1 to 4, 0.1 mL of the liquid was taken out and applied to an M40Y medium sterilized in a petri dish having a diameter of 90 mm. Application was conducted for two media for each sample, and culturing was conducted at 25°C for 7 days. The M40Y medium is a medium that can preferably culture each of xerophilic fungi, xerophilous fungi and hygrophilous fungi, and was obtained by adding to 1 L of distilled water 20g of malt extract, 5g of yeast extract, 400g of sucrose and 20g of agar.

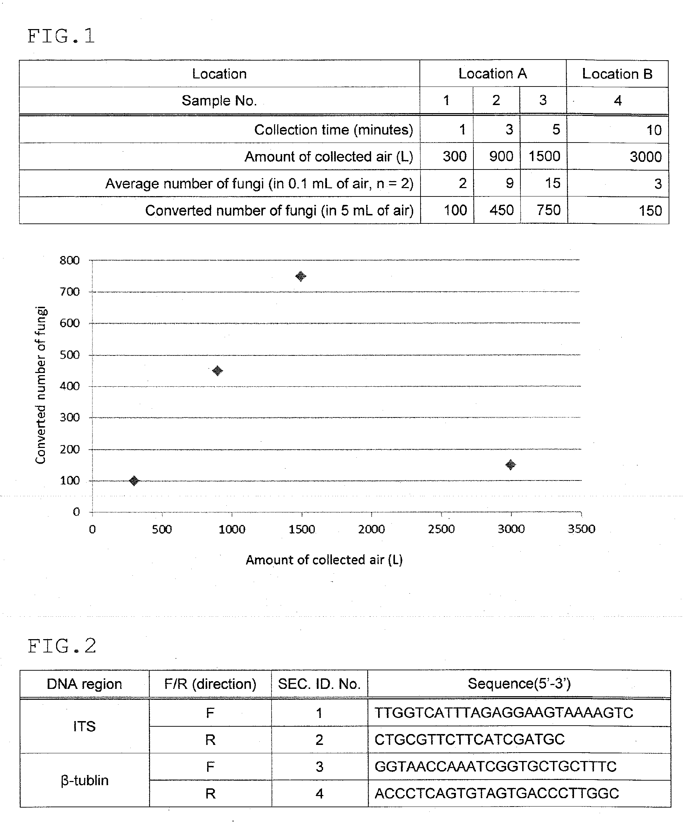

In each of the media, the sample was taken out on the 4^{th} day and the 7^{th} day after the start of culturing and the number of colonies that had been grown was counted visually. The average number of colonies was calculated for each sample. The results are shown in Fig. 1.

As shown in Fig. 1, the average number of fungi in 0.1 mL of samples 1 to 4 was 2, 9, 15 and 3, respectively. Therefore, the number of fungi in 5 mL of the liquid is converted to 100, 450, 750 and 150, respectively. Taking the amount of air collected into consideration, the number of fungi in samples 1 to 3 at the location A was larger than the number of fungi in sample 4 at the location B. The reason therefor is assumed that the air at the location A is contaminated as compared with the air at the location B.

Subsequently, in the nucleic acid extraction step, by using a commercially available nucleic acid extraction kit (Water RNA/DNA Purification Kit-0.22 µm, manufactured by Norgen Biotek Corporation), an operation was conducted in accordance with a manual, whereby a nucleic acid was extracted from the fungi in the liquid of each of samples 1 to 4.

At this time, 5 mL of the liquid of each of samples 1 to 4 was filtered by means of a filter, and then the filter was separated. After collecting the filter in a crushing tube containing glass beads, a bacteriolysis buffer was added, and crushing was conducted at 4800 rpm for 5 minutes. Fungi that had been collected in the liquid were crushed by means of the glass beads together with the filter by this step, whereby a predetermined bacteriolysis treatment was conducted. In a solution obtained by this treatment, the cell walls of the spores and the mycelia of the fungi that had been collected from the air to the liquid were crushed, whereby the bacteriolysis was conducted.

For each of samples 1 to 4, the resulting solution was purified to obtain a DNA solution.

Subsequently, in the nucleic acid amplification step, for each of samples 1 to 4, part of DNA in the DNA solution was amplified by the PCR method.

At this time, as the fungi to be detected, *Aspergillus penicillioides, Cladosporium sp.* and *Wallemia sebi* were selected.

As shown in Fig. 2, as a primer set to amplify the ITS region in the genomic DNA of each of these three types of fungi, a forward primer formed of a base sequence shown by the SEC ID No. 1 and a reverse primer formed of a base sequence shown by the SEC ID No. 2 were used. Further, as a primer set to amplify the β-tubulin region in the genomic DNA of *Aspergillus penicillioides*, a forward primer formed of a base sequence shown by the SEC ID No. 3 and the reverse primer formed of a base sequence shown by the SEC ID No. 4 were used.

As the PCR reaction liquid, by using Ampdirect (R) (manufactured by Shimadzu Corporation), 20 µl of a PCR solution having the following composition was prepared for each sample.
1. Ampdirect addition (G/Crich) 4.0 µl
2. Ampdirect (addition-4) 4.0 µl
3. dNTPmixture 1.0 µl
4. 1.0 mM Cy5-dCTP 0.2 µl
5. Forward primer for amplifying ITS1 region (2.5 µM, SEC ID No. 1, synthesized by Sigma-Aldrich) 1.0 µl
6. Reverse primer for amplifying ITS1 region (2.5 µM, SEC ID No. 2, synthesized by Sigma-Aldrich) 1.0 µl
7. Forward primer for amplifying β-tublin region (10 µM, SEC ID No. 3, synthesized by Sigma-Aldrich) 1.0 µl
8. Reverse primer for amplifying β-dublin (10 µM, SEC ID No. 4, synthesized by Sigma-Aldrich) 1.0 µl
9. Nova Taq Hot Start DNA polymerase 0.2 µl
10. Genomic DNA of a specimen 1.0 µl
11. Water (added until the total quantity became 20.0 µl)

By using the PCR reaction liquid, DNA was amplified for each sample by means of a nucleic acid amplification apparatus (TaKaRa PCR Thermal Cycler Dice (R) Gradient manufactured by Takara Bio Inc.).
1. 95°C 10 minutes
2. 95°C 30 seconds
3. 56°C 30 seconds
4. 72°C 60 seconds (2 to 4 are repeated 40 cycles)
5. 72°C 10 minutes

Subsequently, in the detection step, a DNA chip on which a probe for detecting *Aspergillus penicillioides,* a probe for detecting *Cladosporium sp.* and a probe for detecting *Wallemia sebi* were immobilized was used for each sample. As the DNA chip, a gene silicon (R) (manufactured by Toyo Kohan Co., Ltd.) was used.

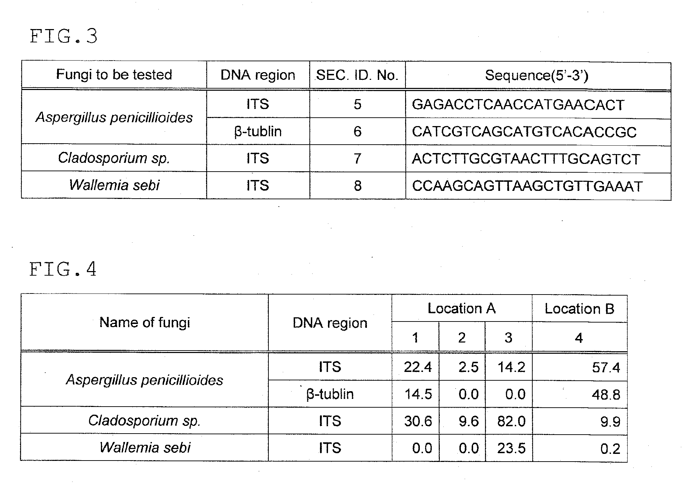

Further, as shown in Fig. 3, as the probe for detecting *Aspergillus penicillioides, a* probe formed of a base sequence shown by the SEC ID No. 5 or 6 was used, as the probe for detecting *Cladosporium sp.* a probe formed of a base sequence shown by the SEC ID No. 7 was used, and as the probe for detecting *Wallemia sebi,* a probe formed of a base sequence shown by the SEC ID No. 8 was used.

The probe formed of a base sequence shown by the SEC ID No. 5, 7 or 8 hybridizes part of a region to be amplified of a primer set for amplifying the ITS region. Further, a probe formed of a base sequence shown by the SEC ID No. 6 hybridizes part of a region to be amplified of a primer set for amplifying the β-tublin region.

A buffer liquid (3 × SSC + 0.3%SDS) was mixed with an amplified product obtained by the PCR method and the resulting was dropped to the DNA chip.

This DNA chip was allowed to stand at 45°C for 1 hour, and a PCR product that had not been hybridized was washed away from the DNA chip by using the above-mentioned buffer liquid.

Subsequently, the DNA chip was mounted in a label detector (BIOSHOT, gene silicon-detected scanner, manufactured by Toyo Kohan Co., Ltd.), and the fluorescence intensity in each probe was measured. The results are shown in Fig. 4.

In Fig. 4, each numerical value shows an S/N ratio ((fluorescence intensity value - background value)/background value). If the value is 3 or more, it can be judged that fungi are present. As for the *Aspergillus penicillioides,* when the S/N value is 3 or more for both the ITS region and the β-tublin region, the fungi are judged to be present. As a result, occurrence of a false positive reaction can be appropriately prevented, thus enabling a more accurate testing to be conducted.

As shown in Fig. 4, for the location A, presence of *Aspergillus penicillioides* could be confirmed by sample 1. Further, by samples 1 to 3, presence of *Cladosporium sp.* could be confirmed. By sample 3, presence of *Wallemia sebi* could be confirmed.

For the location B, by sample 4, presence of *Aspergillus penicillioides* and presence of *Cladosporium sp.* could be confirmed.

As mentioned above, it has been revealed that, according to the method for testing a microorganism in this embodiment, it becomes possible to detect a microorganism without conducting pre-culturing of the microorganism. Further, the time required for sampling was only 10 minutes, whereby the time required for the entire testing from sampling to detection of a microorganism could be significantly shortened.

As a result, according to the method for testing a microorganism in this embodiment, the results of the testing can be obtained on the same day where a specimen is obtained by sampling from the environment, and the testing period that conventionally required several days including sampling can be significantly shortened. Further, from the above-mentioned results, it has been revealed that a testing can be conducted for 750 less, and further 450 or less, 150 or less and 100 or less of fungi in terms of converted number.

The present invention is not limited to the above-mentioned embodiments or examples, and it is needless to say that various modifications are possible within the scope of the present invention.

For example, although in the above-mentioned examples, only three types of fungi were used as a target for testing, it is needless to say that, due to the features thereof, the present invention can be applied irrespective of the type of a microorganism, and appropriate modifications are possible, e.g. a similar method is used for detecting other types of fungi or other microorganisms.

### Industrial Applicability

The present invention can be preferably used for testing plural types of fungi simultaneously in food inspection, epidemiological environmental testing, environmental testing, clinical studies, livestock hygiene, or the like.

## Claims

1. A method for testing a microorganism to simultaneously judge the presence or absence of plural types of microorganisms in the environment, which comprises:
a sampling step for collecting microorganisms floating in the air in a liquid;
a nucleic acid extraction step for extracting nucleic acids from the microorganisms in the liquid;
a nucleic acid amplification step for amplifying part of the nucleic acids extracted; and
a detection step for dropping a solution containing the amplified nucleic acids to a carrier on which nucleic acids of plural types of microorganisms have been immobilized in advance and identifying the types of microorganisms when nucleic acids that bind complementary with the amplified nucleic acids are immobilized on the carrier, wherein
culturing of the microorganisms is not conducted after the sampling step and before the nucleic acid extraction step.

2. The method for testing a microorganism according to claim 1, wherein the microorganism is fungus in the form of a spore or mycelium.

3. The method for testing a microorganism according to claim 1 or 2, wherein the microorganism includes corpses of thereof and the type of the microorganism as the corpse is identified in the detection step.

4. The method for testing a microorganism according to any one of claims 1 to 3, wherein air is collected in an amount of 300 to 3000L by using an air sampler in the sampling step, and a microorganism floating in the collected air is collected in a liquid.
